Europäisches Patentamt

European Patent Office

Office européen des brevets

(19)

(11) Numéro de publication: **0 351 301 B1**

(12) **FASCICULE DE BREVET EUROPEEN**

(45) Date de publication de fascicule du brevet: **24.11.93**

(51) Int. Cl.⁵: **A61K 9/02**, A61K 47/34, A61K 31/575, A61K 9/70

(21) Numéro de dépôt: **89401979.3**

(22) Date de dépôt: **10.07.89**

(54) **Composition pharmaceutique pour la prévention des maladies sexuellement transmissibles.**

(30) Priorité: **11.07.88 FR 8809418**

(43) Date de publication de la demande:
**17.01.90 Bulletin 90/03**

(45) Mention de la délivrance du brevet:
**24.11.93 Bulletin 93/47**

(84) Etats contractants désignés:
**AT BE CH DE ES FR GB GR IT LI LU NL SE**

(56) Documents cités:
EP-A- 0 081 370      EP-A- 0 113 998
EP-A- 0 255 902      EP-A- 0 285 285
FR-A- 2 157 784      FR-A- 2 614 525
GB-A- 1 161 484      GB-A- 1 185 943
GB-A- 2 153 686

Fiche Commerciale de DOW CORNING, 365
Medical Grade Emulsion, Bulletin No. 51-747,
mars 1985

(73) Titulaire: **SOCIETE ANONYME S S P L SAFE SEX PRODUCTS LICENSING**
**6 Rue Monceau**
**Paris 8ème(FR)**

(72) Inventeur: **Augros, Jacques, Julien**
**Chemin de la Croix Baillet**
**Villiers Le Bel Val D'Oise(FR)**

(74) Mandataire: **Gillard, Marie-Louise et al**
**Cabinet Beau de Loménie**
**158, rue de l'Université**
**F-75340 Paris Cédex 07 (FR)**

EP 0 351 301 B1

## Description

La présente invention est relative à la prévention des maladies sexuellement transmissibles telles que, notamment, le SIDA et dénommées ci-après par leur abréviation usuelle "MST".

Les MST les plus courantes sont dues notamment à des virus, bactéries, organismes parasitaires et champignons. Face au développement de ces maladies, il n'existe à ce jour aucun moyen de prévention d'une efficacité suffisante et susceptible d'offrir une alternative à l'utilisation des préservatifs dont on connaît, pourtant, les imperfections et les inconvénients.

En effet, on sait que différents agents chimiques possédent une certaine efficacité sur des germes responsables des MST. Parmi les agents les plus couramment utilisés, on peut citer le chlorure de diméthylalkylbenzalkonium ou chlorure de benzalkonium et le nonylphénolpolyoxyéthylène ou nonoxynol 9.

Toutefois, ces produits ne présentent qu'une efficacité relative en ce sens que, d'une part, leur spectre d'action est étroit puisqu'il ne couvre que quelques uns des germes responsables des MST et, d'autre part, le délai nécessaire à leur action est long et de ce fait inacceptable lorsqu'il s'agit de la prévention des MST.

On sait par exemple que la contamination par le virus du SIDA peut survenir dès les toutes premières minutes de son contact avec des muqueuses saines. En revanche, l'inactivation de ce virus par le chlorure de benzalkonium à une concentration de 1 % n'intervient qu'après un temps de contact d'au moins dix minutes, ce qui laisse une possibilité de contamination extrêmement importante et donc inacceptable en pratique.

Par conséquent, l'utilisation des produits connus ne permet pas une prévention efficace des MST, même lorsqu'ils possèdent des propriétés virucides.

Le brevet EP-A-0 255 902 décrit une composition pour usage topique ayant une activité spermicide, virucide et désinfectante qui comprend un véhicule partiellement préhydraté capable d'absorber des fluides biologiques.. Cette absorption de fluides biologiques limite les risques de contamination par les virus qu'ils pourraient contenir.

Par ailleurs, le brevet GB-A- 1 161 484 décrit des compositions désodorisantes et désinfectantes utilisables en hygiène féminine.Il ne concerne pas des compositions destinées à la prévention des MST. Les compositions décrites dans ce document contiennent un agent bactéricide et un agent émollient qui peut, entre autres, être une huile de silicone.

On sait, par ailleurs, selon le document EP-A-0 113 998, que des dérivés de l'acide cholique : acides déoxycholique et déhydrocholique principalement, peuvent être utilisés dans le traitement des infections virales causées par le virus de l'Herpès Simplex I ou II.

Cependant, il s'agit là d'une action thérapeutique et non préventive alors que le propos de la présente invention est, précisément, de fournir un moyen de prévention. La frontière entre la thérapie et la prévention n'est pas toujours très nette, mais la compréhension de la différence entre ces deux modes d'action est ici essentielle, car c'est le choix de l'un ou de l'autre qui conditionne la détermination de la composition pharmaceutique utilisée, la mise en oeuvre du traitement, sa forme, son mode d'administration ainsi que l'étendue de son utilisation. A la différence de l'action préventive qui concerne des sujets sains, l'action thérapeutique, telle celle envisagée dans le brevet EP-A-0 113 998, produit ses effets sur un sujet déjà atteint. Dans ce cas, c'est lorsque l'infection est visible, ou pour le moins décelable, et par conséquent bien établie, que le malade peut recourir à un remède. Il est donc possible de déterminer très précisément les endroits infectés par le virus de l'Herpès Simplex I ou II, là où devra être appliqué le traitement, si l'on utilise la voie topique.

En effet, deux modes d'administration sont envisageables : la voie parentérale ou la voie topique au sens d'une application très localisée sur le lieu précis où se situe l'infection.

Dans la présente invention, une administration par voie topique signifie l'application d'une composition pharmaceutique non pas sur une zone strictement délimitée par une infection déclarée mais au contraire sur une vaste zone, souvent complexe et toujours imprécise puisque l'infection que l'on veut prévenir est, par conséquent, non localisée.

Ainsi, l'expression "par voie topique" utilisée dans le cas de la thérapie et donc dans le brevet EP-A-0 113 998 (traitement d'un point précis déjà atteint) ne doit pas être confondue avec la même expression employée ici dans le cas de la prévention, dont la signification, indiquée ci-dessus, est tout à fait différente.

Par ailleurs, dans l'objetif de la prévention des MST, la voie topique est préférée à la voie parentérale en raison de la présence de certains composés nécessaires à une réelle efficacité mais qui ne doivent pas être introduits dans l'organisme. Ils peuvent, en effet, devenir toxiques lorsqu'ils sont absorbés, à des dosages importants, puis métabolisés par l'organisme.

Il faut noter, enfin, que dans le brevet EP-A-0 113 998, seule l'action sur le virus de l'Herpès Simplex I et II a été décrite, alors que la présente invention est relative à la prévention contre un spectre étendu de

EP 0 351 301 B1

virus responsables des MST dont les principales souches sont, pour exemples :
- les virus de l'Herpès génital I et II,
- les virus du SIDA HIV I et HIV II,
- le virus de l'hépathite B,
- le papillomasvirus (HPV).

A cette fin, la présente invention implique la protection de vastes zones complexes.

Or, l'enseignement du Brevet EP-A-0 113 998, n'incite pas l'homme de métier à utiliser l'un des dérivés de l'acide cholique :
- d'une part, pour une action préventive efficace pendant plusieurs heures qui nécessite l'application d'une composition pharmaceutique, de façon uniforme et suffisante, sur une zone mal délimitée soit saine mais susceptible d'être contaminée, soit atteinte et donc susceptible de contaminer un partenaire,
- d'autre part, pour un spectre étendu de virus qui correspond à des manifestations différentes de celles de l'Herpès Simplex et donc de portée beaucoup plus générale.

Ainsi, la présente invention concerne une composition pharmaceutique contenant un ou plusieurs principes actifs, efficace quant à la prévention des MST.

La présente invention concerne également une composition pharmaceutique permettant la protection, par voie topique, de zones à haut risque telles que le vagin de la femme.

Le vagin est une muqueuse qui, par nature, favorise le passage de substances aussi bien par émission vers l'extérieur d'elle-même que par absorption vers l'organisme interne.

Or, pour une prévention efficace des MST, le ou les principes actifs ne doivent pas être absorbés par l'organisme, notamment via une muqueuse vaginale.

En effet, jusqu'à présent on ne pouvait utiliser certaines substances pour une telle prévention, bien qu'elles soient efficaces, puisque ces substances diffusaient rapidement dans l'organisme.

C'est ce problème que résout la présente invention.

A cette fin, l'invention a pour objet une composition pharmaceutique pour la prévention des maladies sexuellement transmissibles, destinée à être mise au contact d'une muqueuse caractérisée en ce qu'elle comporte d'une part au moins un principe actif contre des virus ou bactéries responsables desdites maladies sexuellement transmissibles et d'autre part un produit inhibiteur de pénétration dudit principe actif à travers la muqueuse, en combinaison avec un véhicule pharmaceutiquement acceptable adapté à une administration par voie topique de cette composition.

L'invention sera mieux comprise par la description détaillée ci-après donnée.

L'inhibiteur de pénétration est un agent inerte et atoxique apte à former un film isolant et protecteur sur les muqueuses ou sur la peau.

Par ailleurs, pour être suffisante, la protection doit être effective dès l'orifice vaginal et recouvrir la totalité de la muqueuse, ce qui est très difficile à réaliser en raison des replis extrêmement nombreux de cette dernière.

La composition pharmaceutique selon l'invention contient avantageusement un agent inerte et atoxique dispersant le ou les principes actifs afin d'obtenir un étalement uniforme et suffisant de la composition.

L'agent inhibiteur de pénétration et l'agent dispersant contenus dans la composition selon l'invention sont constitués par un même composé de la famille des silicones.

La composition pharmaceutique conforme à la présente invention contient par exemple un sel de l'acide cholique à titre de principe actif.

On sait que l'acide cholique est un constituant naturel de la bile et est actuellement utilisé en thérapie, ainsi que ses sels pharmaceutiquement acceptables, comme agent cholérétique.

On connaît, selon la demande de brevet EP-A-0 285 285, l'utilisation d'un dispersant présenté dans ce document comme étant "tout agent capable d'abaisser la tension interfaciale en milieu aqueux", en combinaison avec un dérivé de l'acide cholique pour le traitement des infections virales. Sachant que le virus utilise les protéines de la cellule qu'il parasite pour former une coque autour de sa chaine d'acide nucléïque (ADN ou ARN), l'action anti-virale du dispersant, postulée dans la demande de brevet EP-A-0 285 285, réside en la rupture de la coque du virus qui, ne pouvant plus envahir d'autres cellules, est ainsi détruit.

Il est important de préciser ici que le dispersant, dont l'action est indiquée ci-dessus, entre dans une composition pharmaceutique administrée par voie parentérale. La composition pharmaceutique contenant ledit dispersant est injectée directement dans la circulation sanguine.

L'action de l'agent dispersant en combinaison avec un ou plusieurs principes actifs dans la composition pharmaceutique conforme à la présente invention est tout à fait différente.

3

En effet, ici, cet agent n'intervient pas directement sur le virus, contrairement à ce qui est décrit dans la demande de brevet ci-dessus.

La composition selon l'invention est destinée à une administration par voie topique contrairement aux modes d'administration (voie parentérale et voie orale) décrits dans la demande de brevets EP-A-0 285 285.

La qualité principale de l'agent filmogène contenu dans la composition selon l'invention est de former sur toute la surface de la zone que l'on souhaite protéger, un film uniforme et isolant qui joue un rôle de barrière :

- il inhibe la pénétration du principe actif dans l'organisme, évitant à celui-ci d'être métabolisé; après étalement, il laisse le temps d'agir audit principe actif;
- il évite la diffusion des virus responsables des MST à travers la muqueuse. Les virus indésirables sont ainsi maintenus au contact du principe actif qui peut les détruire;
- son rôle isolant et protecteur est double en ce sens que d'une part, il protège une femme saine contre une éventuelle contamination par un partenaire atteint et que d'autre part, dans le cas où la femme est atteinte, il protège le partenaire, de par le même effet isolant.

La combinaison de l'effet filmogène et de l'effet dispersant permet un étalement sur la totalité de la surface des replis constituant une muqueuse vaginale.

Le sel de l'acide cholique utilisé dans la composition pharmaceutique selon l'invention est de préférence le cholate de sodium.

Le cholate de sodium est un puissant agent virucide et bactéricide Ce composé est une substance biologique dépourvue de toxicité et active même à de faibles concentrations. La quantité de cholate de sodium à mettre en oeuvre dans la composition pharmaceutique selon l'invention est avantageusement comprise entre 0,2 et 1,5%, de préférence entre 0,25 et 1% en poids par rapport au poids total de la composition.

L'agent filmogène et dispersant préféré aux fins de l'invention est le diméthylpolysiloxane qui possède aussi un effet anti-adhésif renforçant l'effet isolant du film.

La quantité de diméthylpolysiloxane contenue dans la composition pharmaceutique objet de la présente invention est de l'ordre de 10 % en poids d'une émulsion à 35 % de diméthylpolysiloxane par rapport au poids total de la composition.

On remarque que, selon l'invention, par comparaison avec les quantités de principe actif, la quantité d'agent filmogène est très importante.

Cela est dû au fait que l'on recherche la formation d'un film non seulement sur toute l'étendue de la surface à protéger (qui est grande dans le cas de la muqueuse vaginale) mais également selon une compacité suffisante pour obtenir un effet de barrière isolante aussi efficace que possible et cela à deux fins :

- minorer, (mais on souhaite les empêcher totalement), les risques de transfert d'agents pathogènes aussi bien en provenance qu'en direction de la muqueuse,
- éviter l'absorption par la muqueuse des principes actifs associés au film et, donc, maintenir sur place lesdits principes actifs, là où leur action topique est nécessaire.

Cette quantité peut être de préférence accrue plutôt que diminuée du fait que l'effet filmogène a tendance à augmenter avec la concentration.

Le véhicule pharmaceutiquement acceptable qui convient aux fins de l'invention est un véhicule classique adapté à une administration par voie topique. Dans le cas de la protection des muqueuses vaginales, le véhicule doit convenir à un usage interne et prolongé.

L'homme de métier choisira de préférence un véhicule aqueux qui autorise une adhérence et une dilution avec les fluides naturels présents.

Pour une mise en oeuvre avantageuse, la composition selon l'invention peut contenir, en outre, un agent tampon tel que l'acide chlorhydrique afin de pouvoir ajuster le pH vers 4,7. Cette valeur permet de respecter la flore vaginale et le bacile de Doederlein. La non destruction du bacile de Doederlein est très importante car il possède des propriétés défensives capitales contre les infections génitales. Il est donc nécessaire de le préserver.

La composition pharmaceutique selon l'invention peut se présenter sous la forme de solutions (douches vaginales), de crèmes ou de gels.

L'homme de métier choisira un véhicule pharmaceutiquement acceptable adapté à la présentation retenue.

La forme sous laquelle se présente la composition conforme à l'invention est liée à son mode d'utilisation. Cette composition doit posséder, en outre, une certaine viscosité adaptée à la manière dont elle sera utilisée.

4

A cette fin, la composition pharmaceutique selon l'invention contient, en outre, un agent épaississant permettant d'obtenir le comportement rhéologique convenable.

Parmi les agents épaississants classiques, le composé préféré aux fins de l'invention est l'hydroxypropylméthylcellulose, vendue sous la dénomination commerciale "Métholose 60 SH 4000" par la société SEPPIC, 70 Avenue des Champs-Elysées, 75008 Paris (France).

Cet épaississant permet d'ajuster la viscosité jusqu'au degré le mieux adapté à la forme de la composition (solution, crème, gel, etc.). La viscosité ainsi obtenue n'est pas modifiée de façon notable lorsque le pH de la composition varie au cours de son élaboration.

L'importance de cet agent sera mieux comprise à l'aide de l'application exposée plus loin.

Ainsi, les dosages les plus fréquemment retenus pour une composition pharmaceutique selon l'invention sont résumés dans le Tableau I :

## Tableau I

- 0,25 à 1 % en poids de cholate de sodium
- 10 % en poids d'une émulsion à 35 % de diméthylpolysiloxane
- 4 % en poids d'hydroxypropylméthylcellulose
- acide chlorhydrique : quantité nécessaire pour pH =4,7
- eau : quantité voulue pour 100 %.

Selon un mode de réalisation plus élaboré de l'invention, on combine plusieurs principes actifs notamment pour renforcer l'action du cholate de sodium, en particulier sur certaines baetéries.

Par conséquent, la composition selon l'invention peut contenir un ou plusieurs agents virucides et bactéricides, ayant en outre des propriétés spermicides, en combinaison avec le cholate de sodium et l'agent filmogène dispersant. Parmi ces agents virucides, bactéricides et spermicides, on choisit de préférence le chlorure de benzalkonium et/ou le nonoxynol 9.

On remarque ici que la possibilité d'utiliser le nonoxynol 9 illustre les avantages procurés par l'invention. En effet, le nonoxynol 9 peut développer une certaine toxicité, lorsqu'il pénètre dans l'organisme à des doses importantes, en raison de son accumulation notamment dans les reins. De plus, sa capacité de passage à travers une muqueuse vaginale s'élève à 80% environ. Or, l'utilisation du nonoxynol 9 dans une composition selon l'invention est tout à fait possible et sans risque grâce à la présence du film isolant créé par le filmogène contenu dans cette composition qui est un inhibiteur s'opposant, donc, à la pénétration de ce principe actif dans l'organisme.

Les quantités mises en oeuvre sont comprises de préférence entre 0,5 et 1% et 0,25 et 1% en poids par rapport au poids total de la composition, respectivement pour le chlorure de benzalkonium et le nonoxynol 9.

Ainsi, une composition pharmaceutique qui convient particulièrement aux fins de l'invention contient, de préférence les quantités indiquées dans le tableau II ci-dessous:

## Tableau II

- 0,25 à 1 % en poids de cholate de sodium
- 0,50 à 1 % en poids de chlorure de benzalkonium
- 0.25 à 1 % en poids de nonoxynol 9
- 10 % en poids d'une émulsion à 35 % de diméthylpolysiloxane
- 4 % en poids d'hydroxypropylméthylcellulose
- acide chlorhydrique : quantité nécessaire pour pH =4,7
- eau : quantité voulue pour 100 %.

La composition pharmaceutique élaborée selon les proportions du Tableau II ci-dessus est particulièrement adaptée à la prévention des MST en raison de sa spécificité et permet d'obtenir une protection très efficace.

En effet, son action est à la fois virucide, spermicide et bactéricide.

Elle permet ainsi une inactivation efficace des virus responsables des MST mais également des bactéries et champignons eux aussi responsables des MST, parmi lesquelles on peut citer :

. Staphylococus Aureus,
. Candida Albicans
. Streptococus Agalactiae,
. Neisseria Gonorrhoeae
. Garnella Vaginalis,
. Trichonomas Vaginalis,

Les trois agents actifs combinés dans la composition du tableau II agissent d'une manière complémentaire, et possèdent donc un spectre d'action très large.

On peut citer comme exemple, l'action efficace du nonoxynol 9 sur Chlamidia Trachomatis qui provoque néanmoins une augmentation très importante des infections dues à Candida Albicans. Or, le chlorure de benzalkonium, présent également dans la composition, possède, lui, une action très nette sur Candida Albicans.

Pour la prévention des MST, la composition pharmaceutique selon l'invention peut être avantageusement utilisée en combinaison avec un tampon vaginal tel que celui décrit par l'inventeur dans le brevet FR-A-2 614 525. Ce tampon remédie aux inconvénients inhérents aux tampons classiques employés en contraception : difficulté d' extraction, irritation, manque de discrétion. Cela résulte de la présence, sur son corps, d'au moins un évidement qui sert de dispositif d'accrochage et d'extraction.

Pour une mise en oeuvre avantageuse de la composition pharmaceutique conforme à la présente invention, le tampon doit posséder, par ailleurs, des caractéristiques relatives à sa composition et à son mode de préparation.

Ainsi, un tampon utilisé comme support de la composition pharmaceutique selon l'invention, pour la prévention des MST, est constitué d'une mousse de type à cellules ouvertes telle que par exemple une mousse de polyuréthanne éther.

La mousse de polyuréthanne éther constituant un tampon utilisé aux fins de l'invention possède une densité comprise de préférence entre 15 et 28.

D'autre part, la mousse de polyuréthanne éther présente une résistance à la rupture comprise de préférence entre 70 à 150 kilo Pascal (KPa).

Enfin, la dimension moyenne des cellules contenues dans la mousse de polyuréthanne éther est comprise de préférence entre 0,67 et 0,53 millimètres (mm).

Pour une mise en oeuvre avantageuse de l'invention, le tampon est préparé de manière à ne pas comporter de "peau" c'est-à-dire une surface extérieure plus ou moins continue et pratiquement démunie de cellules ouvertes.

Pour ce faire, le tampon est découpé dans une plaque d'épaisseur convenable, elle-même découpée dans un bloc de mousse auparavant débarrassé de sa "peau" extérieure par une opération de sciage.

Il appartient à l'homme de métier de choisir, compte tenu des critères exposés précédemment, la forme et les dimensions du tampon adaptées à l'anatomie de l'utilisatrice.

La composition pharmaceutique selon l'invention se présente, de preférence sous forme d'un gel aqueux; aqueux en raison de la partie du corps où le tampon est utilisé. En effet, il s'agit d'un usage interne dans une zone très sensible : la muqueuse vaginale, qui ne doit pas être irritée ou abîmée. D'autre part, cette forme gel est parfaitement adaptée à la rétention dans le tampon. En effet, la composition ne doit pas, par exemple, sortir trop abondamment du tampon lors de la pression exercée sur celui-ci par l'utilisatrice pour l'introduire dans le vagin. Le tampon devant, par ailleurs, être conservé et surtout demeurer efficace pendant un temps assez long, pour le moins plusieurs heures, la forme de la composition pharmaceutique qui imprègne le tampon doit être telle qu'il n'y ait pas une excrétion excessive de celle-ci dans les premières minutes de son application. L'utilisation d'un gel est importante car elle remédie à cet inconvénient qui altérerait l'efficacité. Cette forme gel permet également, en raison de sa viscosité particulière, un bon contact avec les muqueuses ainsi qu'un étalement uniforme et suffisant sur celles-ci.

Ce qui précède souligne également l'importance du rôle de l'épaississant quant à la viscosité nécessaire que doit avoir le gel dont est imprégné le tampon.

La quantité de gel aqueux conforme à l'invention nécessaire pour imprégner un tampon dépend des dimensions et de la composition de celui-ci. Cependant, l'expérience montre qu'il est préférable que le tampon ne soit pas totalement imprégné car d'une part, il y aurait excrétion d'une grande quantité de gel lorsque l'utilisatrice comprime le tampon afin de l'introduire dans le vagin, et d'autre part, il faut laisser aux fluides naturels la possibilité d'être absorbés (par substitution) et d'être neutralisés. La quantité de gel nécessaire pour imprégner le tampon s'avère répondre aux conditions ci-dessus quand elle est de l'ordre de 25 % par rapport au volume d'absorption totale du tampon.

Chaque tampon est emballé individuellement dans une capsule étanche.

Pour un tampon de 45 mm de diamètre et 20 mm d'épaisseur, on choisit une capsule ayant un diamètre intérieur de 46 mm et une hauteur de 24 mm. Le jeu ainsi disponible convient au mode d'imprégnation conforme à l'invention.

La capsule possède un rebord périphérique de 5 mm de large afin de permettre l'operculage de la capsule par soudure d'un couvercle sur ledit rebord.

L'operculage est facilité par la présence du petit espace libre subsistant au-dessus du tampon du fait de la différence des dimensions de la capsule (24 mm) et du tampon (20 mm).

La capsule peut être constituée d'un matériau du type polyvinyle. L'opercule, quant à lui, est constitué d'un complexe d'aluminium classiquement utilisé pour l'operculage de contenants en plastique.

Conformément à l'invention, l'imprégnation est réalisée de la façon suivante :

La quantité nécessaire de gel est disposée au fond de la capsule. Pour un tampon de 45 X 20 mm, il faut environ 5 gramme de gel. Celui-ci se répand alors selon une fine couche sur toute la surface de la capsule.

Le tampon est ensuite posé sur ce gel et la capsule est operculée.

Le gel pénètre dans le tampon par capillarité.

Le temps d'imprégnation est de l'ordre de trente secondes alors que l'operculage est effectué avantageusement en quelques secondes, tout de suite après le dépôt du tampon, c'est-à-dire avant que l'imprégnation proprement dite du tampon soit terminée.

Par conséquent l'imprégnation s'achève pendant les opérations de conditionnement ultérieures. En d'autres termes, l'imprégnation est obtenue "en temps masqué".

La démarche inverse qui consisterait à déposer le gel sur le tampon et non dans le fond de la capsule, est déconseillée si, comme cela est logique, le volume intérieur de la capsule est proche du volume extérieur du tampon. Dans ce cas, en effet, il y a de fortes probabilités pour que du gel soit accidentellement entraîné sur le bord de la capsule lors de son operculage. Comme l'opercule doit être fixé sur ce bord, généralement par soudure, l'interposition de gel constituerait un empêchement à cette soudure et, cela, d'autant plus gravement que le gel contient un silicone. Il suffirait d'une quantité insignifiante de gel pour provoquer un operculage imparfait, c'est-à-dire non étanche.

Par ailleurs, il n'est pas indispensable de stériliser le tampon du fait de sa haute teneur en agents virucides et bactéricides.

Ainsi, l'utilisation d'un tampon imprégné de plusieurs principes actifs combinés présente, du fait de l'activité complémentaire de ceux-ci, des avantages nouveaux et spécifiquement adaptés à la prévention des MST.

En effet, muni de cellules ouvertes sur la totalité de sa surface, le tampon permet au gel aqueux dont il est imprégné d'être présent et actif dès l'application du tampon en vue de son introduction dans l'orifice vaginal et de recouvrir peu à peu la totalité de la muqueuse vaginale au fur et à mesure de sa progression, conditions impératives pour assurer une protection véritable et instantanée.

La fraction de gel aqueux extraite du tampon lors de sa progression est remplacée à la périphérie du tampon par le gel initialement contenu dans les cellules de sa zone centrale grâce aux communications entre les cellules ouvertes. De plus, en raison de ce transfert interne, la quantité de gel sortie est plus importante lors des rapports sexuels, du fait des mouvements qu'ils supposent, ce qui est particulièrement favorable à la destruction des germes contenus dans le milieu vaginal ainsi que dans le sperme et dans les spermatozoïdes.

L'étalement physique du gel présente donc l'avantage d'être constant et régulier, ce qu'il n'est pas possible d'obtenir avec un tampon ordinaire. Ceci vient renforcer l'action du diméthylpolysiloxane qui permet, parallèlement à ses propriétés dispersantes, la formation d'un film protecteur sur les muqueuses et la peau. Un effet isolant et hydrophobe lui sont associés du fait de la nature même du diméthylpolysiloxane qui appartient à la famille des silicones.

Etant donné que le gel a une faible concentration en principes actifs et que l'agent filmogène a un effet inhibiteur de pénétration de ces principes dans l'organisme, le gel conforme à l'invention peut faire l'objet d'un usage répétitif et prolongé.

Ainsi, l'utilisatrice peut renouveler le tampon imprégné aussi souvent qu'elle le souhaite. Un même tampon peut être laissé en place pendant plusieurs heures sans risque de produire une irritation de la muqueuse vaginale, même en cas d'oubli.

D'ailleurs, il est recommandé de le garder au moins pendant six heures après le dernier rapport sexuel pour assurer la meilleure protection possible.

La présence de chlorure de benzalkonium dans la formule selon le tableau II vient renforcer l'action du cholate de sodium existant et évite le risque de "Toxic Shock Syndrome"

Le "Toxic Shock Syndrome" résulte d'une prolifération rapide de Staphylococus Aureus quand d'autres agents pathogènes sont détruits. La gravité de ce développement provient du fait que Staphylococus Aureus engendre des toxines violentes qui deviennent mortelles très rapidement. Or, le chlorure de benzalkonium, comme le cholate de sodium, possède une action marquée sur ce germe très dangereux.

Le tampon imprégné tel que celui décrit précédemment procure une double protection. Pour le SIDA, par exemple, plusieurs cas sont envisageables :

- Rapport sexuel entre une femme saine et un partenaire à maladie déclarée ou séropositif : l'organisme féminin est isolé et protégé par le film recouvrant la muqueuse vaginale. Les principes actifs détruisent les agents infectieux contenus dans le sperme ou amenés par le partenaire.

- Rapport sexuel entre une femme à maladie déclarée ou séropositive et un partenaire sain : le recouvrement de la cavité vaginale par le film de gel isole la muqueuse et les points de passage possibles des virus (érosions, effractions). Les virus ne peuvent alors pas quitter l'organisme féminin atteint. Le gel éjecté du tampon se fixe également sur la verge du partenaire homme, ainsi, le film le protège par contact direct et par effet de rémanence sur la peau.

- Rapport sexuel entre une femme et un partenaire tous deux à maladie déclarée ou séropositifs : l'action du gel évite la réinfection grave des séropositifs, par isolement réciproque et destruction des agents infectieux des deux provenances.

Ce tampon imprégné est donc particulièrement adapté à la prévention contre le SIDA.

Le tampon imprégné tel que celui décrit précédemment peut être employé pour la prévention des MST, en plus des moyens de contraception classiques (pilules, stérilets etc.). Il peut agir, notamment, sans risque de déplacer les fils de certains stérilets.

Il présente également l'avantage d'être discret. En effet, à l'inverse du préservatif, il n'est pas perçu par le couple. Ce facteur est, dans le cas du préservatif, très limitatif quant au développement de son utilisation. En ce qui concerne la présente invention, cet obstacle est supprimé.

De plus, le tampon imprégné est disponible dans un emballage à usage unique et déchirable qui permet de le protéger et de le conserver correctement, et lui confère ainsi une grande facilité d'emploi.

L'application décrite précédemment constitue une utilisation particulièrement privilégiée de la composition pharmaceutique selon l'invention, notamment mais non exclusivement, pour lutter contre la propagation du SIDA.

L'invention va maintenant être décrite plus en détail à l'aide des exemples suivants:

EXEMPLES

Produits utilisés :

- Cholate de sodium vendu par la Société CIPEC, 7 rue Lincoln, 75008 Paris (France).
- Chlorure de benzalkonium vendu sous la dénomination commerciale "REWOQUAT B 50" par la Société SCHERING, 5 rue Le Corbusier, 94150 Rungis (France).
- Nonoxynol 9 vendu sous la dénomination commerciale "SIMULSOL 930 NP" par la Société SEPPIC, 70 Avenue des Champs-Elysées, 75008 Paris (France).
- Diméthylpolysiloxane vendu sous la dénomination commerciale "365 MEDICAL GRADE EMULSION" par la Société DOW CORNING CORPORATION, Midland, Etat du Michigan 48640 (Etats Unis d'Amérique).
- Hydroxypropylméthylcellulose vendue sous la dénomination commerciale "METHOLOSE 60 SH 4000" par la Société SEPPIC, 70 Avenue des Champs-Elysées, 75008 Paris (France).

EXEMPLE I: Tampon vaginal imprégné de gel aqueux selon la formule du Tableau I

Tampon:

- Il est constitué d'une mousse de polyuréthanne éther qui possèdent les caractéristiques suivantes :
  . densité : environ 22
  . résistance à la rupture: environ 130 KPa
  . dimension moyenne des cellules: 0,6 mm.
- Il mesure 45 mm de diamètre et 20 mm d'épaisseur.
- Il possède deux évidements, comme décrit dans le brevet FR-A-2 614 525.

Gel:

- 5 grammes de gel sont nécessaires pour imprégner un tampon possèdant les caractéristiques ci-dessus. (Le volume d'absorption total d'un tel tampon est de 20 grammes.)
- La composition du gel utilisé est la suivante :

| | |
|---|---|
| . Cholate de sodium | 0,025 g (0,5 %) |
| . Diméthylpolysiloxane (solution à 35 %) | 0,50 g (10 %) |
| . Hydroxypropylméthylcellulose | 0,20 g ( 4 %) |
| . Acide chlorhydrique (1N) | QSP pour pH = 4,7 |
| . Eau purifiée | QSP pour 5 g |

Préparation du gel:
(ce mode opératoire est utilisable quelles que soient les quantités mises en oeuvre)

- Dans une cuve en acier inoxydable (Cuve B), le cholate de sodium est dissous, à température ambiante, dans la moitié de la quantité d'eau purifiée. On ajoute ensuite le diméthylpolysiloxane. Cette solution est alors mélangée par agitation jusqu'à obtention d'une solution homogène. Les produits étant tensio-actifs, le mélange doit se réaliser sous une faible vitesse d'agitation.
- Dans une autre cuve en acier inoxydable à double paroi (Cuve A), la moitié de la quantité d'eau purifiée est chauffée à 70°C par circulation de vapeur d'eau dans la double paroi. L'hydroxypropylméthylcellulose est alors introduite et dispersée sous agitation jusqu'à obtention d'un gel homogène. Ce gel est ensuite refroidi à température ambiante par circulation d'eau froide dans la double paroi de la cuve.
- La solution obtenue dans la cuve B est ajoutée progressivement, à température ambiante, au gel préparé dans la cuve A, sous agitation, jusqu'à obtention d'un gel homomgène.
- Le pH est ajusté par addition d'une solution d'acide chlorhydrique 1N en quantité suffisante pour atteindre un pH de 4,7.
- La masse totale du gel est ajustée à 5 grammes par addition d'eau purifiée en quantité suffisante.

Imprégnation du tampon:

- Le gel obtenu ci-dessus est déposé au fond d'une capsule ayant un diamètre intérieur de 46 mm, une hauteur de 24 mm et un rebord périphérique de 5 mm de large.
- Le tampon est posé sur le gel et celui-ci pénètre dans le tampon par capillarité.
- La capsule est operculée par soudure d'un couvercle de manière à la rendre étanche.

EXEMPLE II : Tampon vaginal imprégné de gel aqueux selon la formule du Tableau II.

Tampon :

- Il présente les mêmes caractéristiques que celui de l'exemple I.

Gel :

- 5 grammes de gel sont nécessaires pour imprégner le tampon.
- La composition du gel utilisé est la suivante:

9

| . Cholate de sodium | 0,025 g (0,5 %) |
|---|---|
| . Chlorure de benzalkonium | 0.050 g (1 %) |
| . Nonoxynol 9 | 0.025 g (0.5 %) |
| . Diméthylpolysiloxane (solution à 35 %) | 0,50 g (10 %) |
| . Hydroxypropylméthylcellulose | 0,20 g ( 4 %) |
| . Acide chlorhydrique (1N) | QSP pour pH = 4,7 |
| . Eau purifiée | QSP pour 5 g (4 à 4,2 g) |

Préparation du gel:

- Elle est effectuée de la même manière que pour l'exemple I.
- Le chlorure de benzalkonium et le nonoxynol 9 sont additionnés en même temps que le diméthylpolysiloxane.

Imprégnation du tampon:

- Elle est identique à celle de l'exemple I.

PROPRIETES PHARMACOLOGIQUES

A- Propriétés virucides

Dans une solution infectée avec le virus du SIDA HIV I, on a constaté, par dosage de l'activité de la Transcriptase Inverse, une inhibition de l'agent enzymatique du virus :
a- par le cholate de sodium dans les conditions ci-après :

| Concentration % cholate de sodium | % d'inhibition Transcriptase Inverse | temps de traitement |
|---|---|---|
| 0,04 | 70 | 15 minutes |
| 0,25 | 90 | 5 minutes |
| 0,50 | 99 | 1 minute |

b- par le gel dont la composition correspond à celle de l'exemple II :

| Concentration gel (volume/volume) | % d'inhibition Transcriptase Inverse | temps de traitement |
|---|---|---|
| $10^{-6}$ | 5 | |
| $10^{-5}$ | 7 | |
| $10^{-4}$ | 99 | |
| $10^{-3}$ | 100 | moins de 2 mn |
| $10^{-2}$ | 100 | moins de 2 mn |
| (mn : minute) | | |

B- Propriétés bactéricides

Le gel, dont la composition correspond à celle de l'exemple II présente, à 32 degrés Celsius, l'activité bactéricide suivante :

| Souches | Concentration antiseptique (volume/volume) | Temps de contact nécessaire |
|---|---|---|
| Staphylococus Aureus CIP 53127 | 0,5 % | 5 mn |
| Candida Albicans CIP 1180.79 | 5 % | 5 mn |
| Streptococus Agalactiae CIP 55 118 | 0,5 % | 5 mn |
| Neisseria Gonorrhoeae CIP 79 18 | 0,1 % | 5 mn |
| Garnella Vaginalis CIP 7074 | 0,5 % | 5 mn |

Les souches bactériennes citées dans ce tableau représentent des espèces responsables d'infections vaginales sexuellement transmissibles.

La concentration antiseptique minimale selon la Pharmacopée Française est de 5 % (gramme/millilitre) en 15 minutes.

C- Propriétés antiparasitaires

A la concentration de 0,5 % (gramme/millilitre) en eau avec un temps de contact de 5 minutes à 37 degrés C., le gel (dont la composition correspond à celle de l'exemple II) possède un effet antiparasitaire immédiat qui fait chuter la population de Trichonomas Vaginalis d'un facteur 1000 au moins.

**Revendications**
**Revendications pour les Etats contractants suivants : DE, GB, FR, IT, NL, SE, CH, LI, BE, AT, LU**

1. Composition pharmaceutique pour la prévention des maladies sexuellement transmissibles ("MST") destinée à être mise au contact d'une muqueuse caractérisée en ce qu'elle comporte, d'une part, au moins un principe actif contre des virus ou bactéries responsables desdites MST et, d'autre part, un produit de la famille des silicones sous une forme filmogène destiné à inhiber la pénétration dudit principe actif à travers la muqueuse, en combinaison avec un véhicule pharmaceutiquement acceptable adapté à une administration par voie topique de cette composition.

2. Composition selon la revendication 1, caractérisée en ce que ledit produit de la famille des silicones sous forme filmogène est constitué d'une émulsion dudit silicone.

3. Composition selon la revendication 2, caractérisée en ce que le silicone est le diméthylpolysiloxane.

4. Composition selon la revendication 3, caractérisée en ce qu'elle contient environ 3,5 % en poids de diméthylpolysiloxane.

5. Composition selon l'une des revendications 1 à 4, caractérisée en ce qu'elle contient au moins un sel de l'acide cholique à titre de principe actif.

6. Composition selon la revendication 1, caractérisée en ce qu'elle contient du cholate de sodium.

7. Composition selon la revendication 6, caractérisée en ce qu'elle contient 0,2 à 1,5 % en poids de cholate de sodium.

8. Composition selon la revendication 7, caractérisée en ce qu'elle contient 0,25 à 1 % en poids de cholate de sodium.

9. Composition selon l'une des revendications 1 à 8, caractérisée en ce que le véhicule pharmaceutiquement acceptable est un véhicule aqueux.

10. Composition selon l'une des revendications 1 à 9, caractérisée en ce qu'elle contient, en outre, un agent tampon.

11. Composition selon l'une des revendications 1 à 10, caractérisée en ce qu'elle contient, en outre, un épaississant.

**12.** Composition selon la revendication 11, caractérisée en ce qu'elle contient de l'hydroxypropylméthylcellulose.

**13.** Composition selon l'une des revendications 1 à 12, caractérisée en ce qu'elle contient, en outre, au moins un agent spermicide.

**14.** Composition selon la revendication 13, caractérisée en ce que l'agent spermicide est le chlorure de benzalkonium et/ou le nonoxynol-9.

**15.** Composition selon la revendication 14, caractérisée en ce qu'elle contient 0,5 à 1 % en poids de chlorure de benzalkonium et/ou 0,25 à 1 % en poids de nonoxynol-9.

**16.** Composition selon l'une des revendications 1 à 15, caractérisée en ce qu'elle est utilisée en combinaison avec un tampon vaginal qui possède au moins un évidement qui sert à son introduction et à son extraction.

**17.** Composition selon la revendication 16, caractérisée en ce que le tampon est constitué d'une mousse de type à cellules ouvertes.

**18.** Composition selon la revendication 17, caractérisée en ce que le tampon est constitué d'une mousse de polyuréthane éther possédant une densité comprise entre 15 et 28, une résistance à la rupture comprise entre 70 et 150 KPa et une dimension de cellules comprise entre 0,67 et 0,53 millimètres.

**19.** Composition selon la revendication 18, caractérisée en ce que le tampon est démuni de "peau" en surface afin de présenter des cellules ouvertes sur la totalité de sa surface.

**Revendications pour les Etats contractants suivants : ES, GR**

**1.** Procédé de préparation d'une composition pharmaceutique pour la prévention des maladies sexuellement transmissibles ("MST") destinée à être mise au contact d'une muqueuse caractérisé en ce que l'on mélange, d'une part, au moins un principe actif contre des virus ou bactéries responsables desdites MST et, d'autre part, un produit de la famille des silicones sous une forme filmogène destiné à inhiber la pénétration dudit principe actif à travers la muqueuse, en combinaison avec un véhicule pharmaceutiquement acceptable adapté à une administration par voie topique de cette composition.

**2.** Procédé selon la revendication 1, caractérisé en ce que ledit produit de la famille des silicones sous forme filmogène est constitué d'une émulsion dudit silicone.

**3.** Procédé selon la revendication 2, caractérisé en ce que le silicone est le diméthylpolysiloxane.

**4.** Procédé selon la revendication 3, caractérisé en ce que le diméthylpolysiloxane est présent à environ 3,5 % en poids.

**5.** Procédé selon l'une des revendications 1 à 4, caractérisé en ce que le principe actif est un sel de l'acide cholique.

**6.** Procédé selon l'une des revendications 1 à 5, caractérisé en ce que le principe actif est du cholate de sodium.

**7.** Procédé selon la revendication 6, caractérisé en ce que le cholate de sodium est présent à raison de 0,2 à 1,5 % en poids.

**8.** Procédé selon la revendication 7, caractérisé en ce que le cholate de sodium est présent à raison de 0,25 à 1 % en poids.

**9.** Procédé selon l'une des revendications 1 à 8, caractérisé en ce que le véhicule pharmaceutiquement acceptable est un véhicule aqueux.

**10.** Procédé selon l'une des revendications 1 à 9, caractérisée en ce que l'on ajoute, en outre, un agent tampon.

**11.** Procédé selon l'une des revendications 1 à 10, caractérisé en ce que 'lon ajoute, en outre, un épaississant.

**12.** Procédé selon la revendication 11, caractérisé en ce que l'épaississant est l'hydroxypropylméthylcellulose.

**13.** Procédé selon l'une des revendications 1 à 12, caractérisé en ce que l'on ajoute, en outre, au moins un agent spermicide.

**14.** Procédé selon la revendication 13, caractérisé en ce que l'agent spermicide est le chlorure de benzalkonium et/ou le nonoxynol-9.

**15.** Procédé selon la revendication 14, caractérisé en ce que le chloure de benzalkonium est présent à raison de 0,5 à 1 % en poids et/ou le nonoxynol-9. à raison de 0,25 à 1 % en poids.

**16.** Procédé de préparation d'un tampon vaginal caractérisé en ce qu'il consiste à imprégner un tampon vaginal d'une composition préparée selon le procédé de l'une des revendications 1 à 15.

**17.** Procédé selon la revendication 16, caractérisé en ce que le tampon vaginal vaginal possède au moins un évidement qui sert à son introduction et à son extraction.

**18.** Procédé selon l'une des revendications 16 ou 17 , caractérisé en ce que le tampon est constitué d'une mousse de type à cellules ouvertes.

**19.** Procédé selon la revendication 18, caractérisé en ce que le tampon est constitué d'une mousse de polyuréthane éther possédant une densité comprise entre 15 et 28, une résistance à la rupture comprise entre 70 et 150 KPa et une dimension de cellules comprise entre 0,67 et 0,53 millimètres.

**20.** Procédé selon la revendication 19, caractérisé en ce que le tampon est démuni de "peau" en surface afin de présenter des cellules ouvertes sur la totalité de sa surface.

**Claims**
**Claims for the following Contracting States : DE, GB, FR, IT, NL, SE, CH, LI, BE, AT, LU**

**1.** Pharmaceutical composition for the prevention of sexually transmitted diseases ("STM") intended to be contacted with a mucosa characterized in that it comprises, on the one hand, at least one constituent active against viruses or bacteria responsible for said STM and, on the other hand, a substance of the family of silicones which forms a film intended to inhibit the penetration of said active constituent through the mucosa, in combination with a pharmaceutically acceptable vehicle adapted to a topical administration of said composition.

**2.** Composition according to claim 1, characterized in that said film-forming substance of the silicone family is constituted of an emulsion of said silicone.

**3.** Composition according to claim 2, characterized in that the silicone is dimethylpolysiloxane.

**4.** Composition according to claim 3, characterized in that it contains about 3.5% by weight of dimethylpolysiloxane.

**5.** Composition according to one of claims 1 to 4, characterized in that it contains at least one salt of cholic acid as active constituent.

**6.** Composition according to claim 1, characterized in that it contains sodium cholate.

13

7. Composition according to claim 6, characterized in that it contains 0.2 to 1.5% by weight of sodium cholate.

8. Composition according to claim 7, characterized in that it contains 0.25 to 1% by weight of sodium cholate.

9. Composition according to one of claims 1 to 8, characterized in that the pharmaceutically acceptable vehicle is an aqueous vehicle.

10. Composition according to one of claims 1 to 9, characterized in that it further contains a buffering agent.

11. Composition according to one of claims 1 to 10, characterized in that it further contains a thickening agent.

12. Composition according to claim 11, characterized in that it contains hydroxypropylmethylcellulose.

13. Composition according to one of claims 1 to 12, characterized in that it further contains at least one spermicidal agent.

14. Composition according to claim 13, characterized in that the spermicidal agent is benzalkonium chloride and/or 9-nonoxynol.

15. Composition according to claim 14, characterized in that it contains 0.5 to 1% by weight of benzalkonium chloride and/or 0.25 to 1% by weight of 9-nonoxynol.

16. Composition according to one of claims 1 to 15, characterized in that it is used in combination with a vaginal tampon having at least one recess which serves for its introduction and extraction.

17. Composition according to claim 16, characterized in that the tampon is constituted of a foam of open-cell type.

18. Composition according to claim 17, characterized in that the tampon is constituted of a polyurethane ether foam having a density comprised between 15 and 28, a breaking strength comprised between 70 and 150 KPa and a cell dimension comprised between 0.67 and 0.53 mm.

19. Composition according to claim 18, characterized in that the tampon has no "skin" surface so as to present open cells over the whole of its surface.

**Claims for the following Contracting States : ES, GR**

1. Process for the preparation of a pharmaceutical composition for the prevention of sexually transmitted diseases ("STM") intended to be contacted with a mucosa characterized in that it comprises mixing, on the one hand, at least one constituent active against viruses or bacteria responsible for said STM and, on the other hand, a substance of the family of silicones which forms a film intended to inhibit the penetration of said active constituent through the mucosa, in combination with a pharmaceutically acceptable vehicle adapted to a topical administration of said composition.

2. Process according to claim 1, characterized in that said film-forming substance of the silicone family is constituted of an emulsion of said silicone.

3. Process according to claim 2, characterized in that the silicone is dimethylpolysiloxane.

4. Process according to claim 3, characterized in that the dimethylpolysiloxane is present in the proportion of about 3.5% by weight.

5. Process according to one of claims 1 to 4, characterized in that the active constituent is a cholic acid salt.

14

6. Process according to one of claims 1 to 5, characterized in that the active constituent is sodium cholate.

7. Process according to claim 6, characterized in that the sodium cholate is present in the proportion of 0.2 to 1.5% by weight.

8. Process according to claim 7, characterized in that the sodium cholate is present in the proportion of 0.25 to 1% by weight.

9. Process according to one of claims 1 to 8, characterized in that the pharmaceutically acceptable vehicle is an aqueous vehicle.

10. Process according to one of claims 1 to 9, characterized in that a buffering agent is further added.

11. Process according to one of claims 1 to 10, characterizd in that a thickening agent is further added.

12. Process according to claim 11, characterized in that the thickening agent is hydroxypropylmethylcellulose.

13. Process according to one of claims 1 to 12, characterized in that at least one spermicidal agent is further added.

14. Process according to claim 13, characterized in that the spermicidal agent is benzalkonium chloride and/or 9-nonoxyl.

15. Process according to claim 14, characterized in that the benzalkonium chloride is present in the proportion of 0.5 to 1% by weight and/or the 9-nonoxyl in the proportion of 0.25 to 1% by weight.

16. Process for the preparation of a vaginal tampon, characterized in that it consists in impregnating a vaginal tampon with a composition prepared, according to the process of one of claims 1 to 15.

17. Process according to claim 16, characterized in that the vaginal tampon has at least one recess serving for its introduction and its extraction.

18. Process according to one of claims 16 or 17, characterized in that the tampon is constituted of a foam of open-cell type.

19. Process according to claim 18, characterized in that the tampon is constituted of a polyurethane ether foam having a density comprised between 15 and 28, a breaking strength comprised between 70 and 150 KPa and a cell dimension comprised between 0.67 and 0.53 mm.

20. Process according to claim 19, characterized in that the tampon has no "skin" surface so as to present open cells over the whole of its surface.

**Patentansprüche**
**Patentansprüche für folgende Vertragsstaaten : DE, GB, FR, IT, NL, SE, CH, LI, BE, AT, LU**

1. Pharmazeutische Zusammensetzung zur Vorbeugung gegen sexuell übertragbare Krankheiten ("STD"), die zum Inkontaktbringen mit einer Schleimhaut bestimmt ist, dadurch gekennzeichnet, daß sie einerseits mindestens einen Wirkstoff gegen Viren oder Bakterien, die für die STD verantwortlich sind, und anderseits ein Produkt aus der Gruppe der Silikone in filmbildender Form zur Verhinderung des Eindringens des Wirkstoffs durch die Schleimhaut in Kombination mit einem pharmazeutisch akzeptablen Träger zur Verabreichung dieser Zusammensetzung auf topischem Weg umfaßt.

2. Zusammensetzung nach Anspruch 1, dadurch gekennzeichnet, daß das Produkt aus der Gruppe der Silikone in filmbildender Form aus einer Emulsion dieses Silikons besteht.

3. Zusammensetzung nach Anspruch 2, dadurch gekennzeichnet, daß das Silikon Dimethylpolysiloxan ist.

4. Zusammensetzung nach Anspruch 3, dadurch gekennzeichnet, daß sie etwa 3,5 Gew.% Dimethylpolysiloxan enthält.

5. Zusammensetzung nach einem der Ansprüche 1 bis 4, dadurch gekennzeichnet, daß sie mindestens ein Salz der Cholsäure als Wirkstoff enthält.

6. Zusammensetzung nach Anspruch 1, dadurch gekennzeichnet, daß sie Natriumcholat enthält.

7. Zusammensetzung nach Anspruch 6, dadurch gekennzeichnet, daß sie 0,2 bis 1,5 Gew.% Natriumcholat enthält.

8. Zusammensetzung nach Anspruch 7, dadurch gekennzeichnet, daß sie 0,25 bis 1 Gew.% Natriumcholat enthält.

9. Zusammensetzung nach einem der Ansprüche 1 bis 8, dadurch gekennzeichnet, daß der pharmazeutisch akzeptable Träger ein wässeriger Träger ist.

10. Zusammensetzung nach einem der Ansprüche 1 bis 9, dadurch gekennzeichnet, daß sie außerdem ein Puffermittel enthält.

11. Zusammensetzung nach einem der Ansprüche 1 bis 10, dadurch gekennzeichnet, daß sie außerdem ein Verdickungsmittel enthält.

12. Zusammensetzung nach Anspruch 11, dadurch gekennzeichnet, daß sie Hydroxypropylmethylcellulose enthält.

13. Zusammensetzung nach einem der Ansprüche 1 bis 12, dadurch gekennzeichnet, daß sie außerdem mindestens ein Spermizid enthält.

14. Zusammensetzung nach Anspruch 13, dadurch gekennzeichnet, daß das Spermizid Benzalkoniumchlorid und/oder Nonoxyn-9-ol ist.

15. Zusammensetzung nach Anspruch 14, dadurch gekennzeichnet, daß sie 0,5 bis 1 Gew.% Benzalkoniumchlorid und/oder 0,25 bis 1 Gew.% Nonoxyn-9-ol enthält.

16. Zusammensetzung nach einem der Ansprüche 1 bis 15, dadurch gekennzeichnet, daß sie in Kombination mit einem Vaginaltampon verwendet wird, der mindestens eine Aussparung zu seinem Einbringen und Entfernen besitzt.

17. Zusammensetzung nach Anspruch 16, dadurch gekennzeichnet, daß der Tampon durch einen offenzelligen Schaumstoff gebildet ist.

18. Zusammensetzung nach Anspruch 17, dadurch gekennzeichnet, daß der Tampon durch einen Polyurethanether-Schaumstoff mit einer Dichte zwischen 15 und 28, einer Reißfestigkeit zwischen 70 und 150 kPa und einer Zelldimension zwischen 0,67 und 0,53 mm gebildet ist.

19. Zusammensetzung nach Anspruch 18, dadurch gekennzeichnet, daß der Tampon an der Oberfläche keine "Haut" hat, um über seine gesamte Oberfläche offene Zellen aufzuweisen.

**Patentansprüche für folgende Vertragsstaaten : ES, GR**

1. Verfahren zur Herstellung einer pharmazeutischen Zusammensetzung zur Vorbeugung gegen sexuell übertragbare Krankheiten ("STD"), die zum Inkontaktbringen mit einer Schleimhaut bestimmt ist, dadurch gekennzeichnet, daß man einerseits mindestens einen Wirkstoff gegen Viren oder Bakterien, die für die STD verantwortlich sind, und anderseits ein Produkt aus der Gruppe der Silikone in filmbildender Form zur Verhinderung des Eindringens des Wirkstoffs durch die Schleimhaut in Kombination mit einem pharmazeutisch akzeptablen Träger zur Verabreichung dieser Zusammensetzung auf topischem Weg vermischt.

16

**2.** Verfahren nach Anspruch 1, dadurch gekennzeichnet, daß das Produkt aus der Gruppe der Silikone in filmbildender Form aus einer Emulsion dieses Silikons besteht.

**3.** Verfahren nach Anspruch 2, dadurch gekennzeichnet, daß das Silikon Dimethylpolysiloxan ist.

**4.** Verfahren nach Anspruch 3, dadurch gekennzeichnet, daß das Dimethylpolysiloxan zu etwa 3,5 Gew.% vorhanden ist.

**5.** Verfahren nach einem der Ansprüche 1 bis 4, dadurch gekennzeichnet, daß der Wirkstoff ein Salz der Cholsäure ist.

**6.** Verfahren nach einem der Ansprüche 1 bis 5, dadurch gekennzeichnet, daß der Wirkstoff Natriumcholat ist.

**7.** Verfahren nach Anspruch 6, dadurch gekennzeichnet, daß das Natriumcholat in einer Menge von 0,2 bis 1,5 Gew.% Natriumcholat vorhanden ist.

**8.** Verfahren nach Anspruch 7, dadurch gekennzeichnet, daß das Natriumcholat in einer Menge von 0,25 bis 1 Gew.% vorhanden ist.

**9.** Verfahren nach einem der Ansprüche 1 bis 8, dadurch gekennzeichnet, daß der pharmazeutisch akzeptable Träger ein wässeriger Träger ist.

**10.** Verfahren nach einem der Ansprüche 1 bis 9, dadurch gekennzeichnet, daß man außerdem ein Puffermittel zugibt.

**11.** Verfahren nach einem der Ansprüche 1 bis 10, dadurch gekennzeichnet, daß man außerdem ein Verdickungsmittel zugibt.

**12.** Verfahren nach Anspruch 11, dadurch gekennzeichnet, daß das Verdickungsmittel Hydroxypropylmethylcellulose ist.

**13.** Verfahren nach einem der Ansprüche 1 bis 12, dadurch gekennzeichnet, daß man außerdem mindestens ein Spermizid zugibt.

**14.** Verfahren nach Anspruch 13, dadurch gekennzeichnet, daß das Spermizid Benzalkoniumchlorid und/oder Nonoxyn-9-ol ist.

**15.** Verfahren nach Anspruch 14, dadurch gekennzeichnet, daß das Benzalkoniumchlorid in einer Menge von 0,5 bis 1 Gew.% und/oder das Nonoxyn-9-ol in einer Menge von 0,25 bis 1 Gew.% vorliegt.

**16.** Verfahren zur Herstellung eines Vaginaltampons, dadurch gekennzeichnet, daß es darin besteht, daß ein Vaginaltampon mit einer gemäß dem Verfahren nach einem der Ansprüche 1 bis 15 hergestellten Zusammensetzung imprägniert wird.

**17.** Verfahren nach Anspruch 16, dadurch gekennzeichnet, daß der Vaginaltampon mindestens eine Aussparung zu seinem Einbringen und Entfernen besitzt.

**18.** Verfahren nach einem der Ansprüche 16 oder 17, dadurch gekennzeichnet, daß der Tampon durch einen offenzelligen Schaumstoff gebildet wird.

**19.** Verfahren nach Anspruch 18, dadurch gekennzeichnet, daß der Tampon durch einen Polyurethanether-Schaumstoff mit einer Dichte zwischen 15 und 28, einer Reißfestigkeit zwischen 70 und 150 kPa und einer Zelldimension zwischen 0,67 und 0,53 mm gebildet wird.

**20.** Verfahren nach Anspruch 19, dadurch gekennzeichnet, daß der Tampon an der Oberfläche keine "Haut" hat, um über seine gesamte Oberfläche offene Zellen aufzuweisen.